(19) 

**Europäisches Patentamt**

**European Patent Office**

**Office européen des brevets**

(11) **EP 4 666 955 A1**

(12) **EUROPEAN PATENT APPLICATION**

(43) Date of publication:
24.12.2025 Bulletin 2025/52

(21) Application number: 24183366.4

(22) Date of filing: 20.06.2024

(51) International Patent Classification (IPC):
**A61B 8/00** (2006.01)     **A61B 8/08** (2006.01)

(52) Cooperative Patent Classification (CPC):
**A61B 8/4245; A61B 8/4254; A61B 8/4427; A61B 8/461; A61B 8/5269; A61B 8/54**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
- **Schäfer, Sebastian**
  **96146 Altendorf (DE)**
- **Reichelt, Stefan**
  **96049 Bamberg (DE)**

(74) Representative: **Siemens Healthineers**
**Patent Attorneys**
**Postfach 22 16 34**
**80506 München (DE)**

(54) **ASSISTING AN ULTRASONIC EXAMINATION OF AN OBJECT**

(57) According to a computer-implemented method for assisting an ultrasonic examination of an object (8), imaging data (2) comprising a sequence of ultrasonic images generated by the ultrasonic probe (19) and depicting the object (8) is received during an ultrasonic scan (15) with an ultrasonic probe (19). Besides that, a current position (6) of the ultrasonic probe (19) is monitored during the ultrasonic scan (15). Additionally, the current position (6) of the ultrasonic probe (19) and a predefined target path (5) for the ultrasonic probe (19) are displayed during the ultrasonic scan (15) on a display device (3) to guide an operator of the ultrasonic probe (19). Next to that, the imaging data (2) is classified by applying a trained machine learning model, MLM, to input data depending on the imaging data (2).

FIG 3

**EP 4 666 955 A1**

**Description**

The present invention is directed to a computer-implemented method for assisting an ultrasonic examination of an object. Imaging data comprising a sequence of ultrasonic images generated by an ultrasonic probe and depicting the object is received during an ultrasonic scan. The invention is further directed to an ultrasonic instrument for carrying out such a computer-implemented method and to a corresponding computer program product.

Ultrasonic images are for example integrated images resulting from reflections of ultrasonic waves at transitions of media with different characteristics. In the case the object is a patient, the reflection can occur for example at organ surfaces and/or scattering in heterogeneous tissues. The ultrasonic examination enables non-invasive visualization e.g. of tissue structures in the case that the object is the patient. Ultrasonic scanning is an interactive procedure involving the operator, the object, in particular the patient, and the ultrasonic instrument, which may contain the ultrasonic probe, a display device, a data processing system, cables and other accessories.

Ultrasonic examination is for example being used in acute care to support both diagnosis (e.g. fluid accumulation or foreign bodies) and treatment (e.g. intravenous catheterization or arthrocentesis).

Optimally, ultrasonic examinations should be performed by trained personnel, as the training and experience level of the operator can have a significant influence on the imaging data. Among others, a position of the ultrasonic probe, an elevation angle and an azimuth angle of the ultrasonic probe, an applied pressure on the object, an amount of ultrasonic transmission gel and/or settings of the ultrasonic instrument offer a wide variety of potential incorrect operation and thus unsatisfactory or even unusable imaging data. As the ultrasonic probe may be designed as a hand-held device and the imaging data may directly depend on a perspective of an ultrasonic transducer, which is located at the tip of the ultrasonic probe, results of the ultrasonic examination may not be entirely reproducible and may not necessarily show the desired part of the object, e.g. of the tissue structure.

The generated imaging data may need to be classified, for example to determine deviations from expected results. The classification can be defined as a task of categorizing images into one or multiple predefined classes. Although the task of categorizing an image may be instinctive and habitual to well-trained and/or very experienced personnel, it may be challenging for novice users. Thus, one may for example use an automated system to recognize and classify images. The automated system may be based on a trained machine learning model (MLM).

Several documents including KR102097740B1 describe a system for classifying and standardizing of medical images automatically using artificial intelligence.

It is an objective of the present invention to increase the reliability of a classification of ultrasonic imaging data by a trained machine learning model (MLM).

This objective is achieved by the subject matter of the independent claim. Further implementations and preferred embodiments are subject matter of the dependent claims.

The inventors have recognized that MLM classifications show more reliable results with an increased standardization level of the imaging data. Thus, the invention is based on the idea to implement a standardized workflow including operator feedback in order to overcome potential deficiencies in an examination process, and to classify the imaging data by a trained machine learning model.

According to an aspect of the invention, a computer-implemented method for assisting an ultrasonic examination of an object is provided. Therein, during an ultrasonic scan with an ultrasonic probe, imaging data comprising a sequence of ultrasonic images generated by the ultrasonic probe and depicting the object is received. A current position of the ultrasonic probe is monitored during the ultrasonic scan. The current position of the ultrasonic probe and a predefined target path for the ultrasonic probe, in particular for the ultrasonic probe during the ultrasonic scan, are displayed during the ultrasonic scan on a display device to guide an operator of the ultrasonic probe, in particular to guide the operator to carry out the ultrasonic scan using the ultrasonic probe. The imaging data is classified by applying a trained machine learning model, MLM, to input data depending on the imaging data.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of a method for assisting an ultrasonic examination of an object, which is not purely computer-implemented, is obtained by including respective steps of generating the imaging data, in particular the ultrasonic images, by the ultrasonic probe.

In general terms, a trained MLM may mimic cognitive functions that humans associate with other human minds. In particular, by training based on training data the MLM may be able to adapt to new circumstances and to detect and extrapolate patterns. Another term for a trained MLM is "trained function".

In general, parameters of an MLM can be adapted or updated by means of training. In particular, supervised training, semi-supervised training, unsupervised training, reinforcement learning and/or active learning can be used. Furthermore, representation learning, also denoted as feature learning, can be used. In particular, the parameters of the MLMs can be adapted iteratively by several steps of training. In particular, within the training a certain loss function, also denoted as cost function, can be minimized. In particular, within the training of an artificial neural network, ANN, the backpropagation algorithm can be used.

In particular, an MLM can comprise an ANN, a support vector machine, a decision tree and/or a Bayesian network, and/or the MLM can be based on k-means clustering, Q-learning, genetic algorithms and/or association rules. In particular, an ANN can be or comprise a deep neural network, a convolutional neural network or a convolutional deep neural network. Furthermore, an ANN can be an adversarial network, a deep adversarial network and/or a generative adversarial network, GAN.

Generating the imaging data during the ultrasonic examination may include a manual process but is not necessarily limited to the manual process. In other words, the ultrasonic probe may be positioned and/or guided along the object by hand by the operator. The imaging data may be available as a real-time data stream of a respective part of the object at the current position of the ultrasonic probe. The real-time data stream comprises or corresponds to the sequence of ultrasonic images in this case. The imaging data may be analyzed, for example immediately after their generation, and may also serve as a feedback loop to the operator for changing the current position of the ultrasonic probe, in particular for adapting the current position of the ultrasonic probe to the target path. The process of generating the imaging data is, as mentioned above, is not necessarily part of the claimed computer-implemented method, but can be in some embodiments.

Prior to the ultrasonic scan, for example a picture of the object to be scanned may be taken, in particular of a patient or of a relevant part of the patient. This picture may for example be used as a background outline on the display device. The display device may for example display an overlaid view including the current position of the ultrasonic probe and the predefined target path. During the ultrasonic scan, the operator may hold the ultrasonic probe in one hand and e.g. hold the display device in the other hand or handle another equipment necessary for the ultrasonic scan. The display device may, however, also be mounted or positioned otherwise.

A starting position of the ultrasonic examination may be a predefined, in particular clear and easily recognizable, location on the object, referred to as a landmark here and in the following. In the case of a torso of the patient, the landmark may be for example the navel or the sternum and so forth. The display device may for example instruct the operator to place the ultrasonic probe on the landmark and start the examination along the predefined target path. The display device may for example indicate a progress of the ultrasonic examination.

The current position of the ultrasonic probe may for example be monitored based on sensor data corresponding to a relative or absolute position of the ultrasonic probe. For example, the sensor data may be generated by a sensor system of the ultrasonic probe, for example of at least one accelerometer of the ultrasonic probe. The sensor data may also be generated by an external sensor system, for example a camera observing the ultrasonic probe during the ultrasonic scan. The sensor data may be transmitted from the ultrasonic probe or the external sensor system, respectively, to the data processing system by means of wireless communication or by using a wire-based connection.

The predefined target path may for example correspond to a selection item from a list of several predefined target paths, which may relate to different examination scenarios. In particular, especially such examination scenarios may be typical ultrasonic scanning scenarios. The list of several predefined target paths can be derived from theoretical sources or from prior examinations with lighthouse character. The selection of the predefined target path can be combined with a selection of one or more settings, for example initial settings of an ultrasonic instrument, which can include output power, probe frequency, focus value, and/or gain value of the ultrasonic probe, and/or other ultrasonic instrument parameters. It may be helpful for the overlaid view to scale and/or trim the picture of the object or the predefined target path in order adapt to a different size of the object, in particular size and/or characteristic of the patient.

The overlaid view of the current position in combination with the picture of the object and the predefined target path may serve as a guideline for the operator to place the ultrasonic probe at a necessary next position during the ultrasonic scan. The ultrasonic examination may for example be understood as a continuous process including generating imaging data and placing of the ultrasonic probe at the necessary next position for the duration of the ultrasonic scan.

Classifying the imaging data may for example include assigning a class of two or more predefined classes to the imaging data. The two or more predefined classes may for example indicate whether or not or to which extent the imaging data matches predefined expectations regarding the outcome of the ultrasonic scan, for example for healthy persons or another predefined patient population. For example, the two or more predefined classes may comprise a class indicating that "no significant deviation from expected imaging data is found" and a class indicating that "significant deviation from expected imaging data is found", for example. However, the classification does not necessarily need to be binary but may, in some implementations, be more finely graded.

Prior to classifying the imaging data, image stitching of the ultrasonic images may be carried out. Image stitching may generate a segmented panorama or high-resolution image by a process of combining multiple photographic images with overlapping fields. The process may be applied to the imaging data in order to improve the ultrasonic examination result.

The MLM classification does, in particular, not include an attribution of the deviation to a particular clinical picture, i.e. the deductive medical or veterinary decision phase. In particular, classifying the image data does not include making a medical diagnosis, even though the result of the classification may, in some applications, be used by medically trained personnel to aid a medical diagnosis and/or to decide upon further analysis steps.

The MLM may be a known MLM, in particular ANN, for medical image classification, in particular ultrasonic image classification. The MLM may also be trained using known training methods.

The classification by the MLM may be possible with the imaging data generated along the predefined target path for the selected examination scenario. The classified imaging data may be interpreted or used for further process steps by a data assessor, who does not need to be but may be the operator.

By means of the computer-implemented method according to the invention, the operator is assisted by feedback information, namely feedback information including the current position and the target path, displayed on the display device during the ultrasonic examination. This may lead to shorter examination time and an improved quality level of the imaging data.

In particular, by assisting the operator using the computer-implemented method according to the invention, a significant contribution to standardize the generated imaging data in terms of imaging quality but also in terms of the content of the imaging data may be made. This is beneficial since the trained MLM strongly benefits from said improved standardization and comparability of the imaging data. Thus, the reliability of the classification by the MLM is improved.

In some implementations, the process of the ultrasonic examination and the post-processing of the imaging data is separated. This may lead to a relief of the work burden of the operator and may open the possibility to perform the ultrasonic examination by less trained personnel.

According to several implementations, depending on the current position, a completed path for the ultrasonic probe, in particular an already completed path up to the current position of the ultrasonic probe, is displayed during the ultrasonic scan on the display device.

The completed path may for example be generated by connecting the current position of the ultrasonic probe with the previous positions of the ultrasonic probe, which have been monitored during the ultrasonic scan. In consequence, this may lead to a record of point-to-point connections in chronological order, which is prolonged during the ultrasonic scan. This may, in other words, be a path, which actually has been travelled during the ultrasonic examination. Another possibility may be a comparison between the predefined target path and the path actually travelled in order to show the completed path as part of the predefined target path e.g. in a different color, line style or another differentiating feature. The display device may optionally be understood as a navigation device, which indicates a segment of the predefined target path as actually travelled.

It may also be possible to highlight parts of the object, which have already been successfully examined, as the completed path. To this end, it may be provided that not only the sensor data about current position of the ultrasonic probe is monitored but also a deviation between a content of the imaging data and a predefined reference content is evaluated. The evaluation may include image recognition methods for taking a decision whether or not an expected part of the object was recognized within the content of the imaging data. The predefined reference may reflect the selected examination scenario.

An advantage of such embodiments is that the operator may better observe a progress of the ultrasonic scan and may also get a visual feedback of their action. A novice operator might not be able to reliably find the necessary parts of the object without assistance. In particular, this part might be the organ or any other tissue, which is predefined in the examination scenario. It may therefore be assured, that the operator has perceived the information on the predefined target path and also was able to check their action with a real-time feedback. This may shorten the examination time and increase a hit rate of the desired parts of the object in the imaging data.

According to several implementations, a first warning signal for the operator is generated, if a distance between the current position and the predefined target path exceeds a predefined first threshold value.

In other words, the first warning signal is generated if the current position moved away from the predefined target path by at least the predefined first threshold value. In particular, the distance between the current position and the predefined target path is computed, in particular monitored during the ultrasonic scan, and compared to the first threshold value. If it is found based on the comparison, that the distance exceeds the first threshold value, then the first warning signal is generated.

It may also be possible to monitor a speed of a movement of the ultrasonic probe, for example by computing a position difference over a defined time period. The speed of the movement may for example be limited to a certain maximum speed, which allows a high degree of data recognition for the imaging data.

The operator may receive the first warning signal as a direct feedback to their performance during the ultrasonic examination. The predefined first threshold value may vary depending on the examination scenario, size or characteristic of the object, the training level of the operator or other factors. For example, a small predefined first threshold value may be intended for a narrow tracking of the predefined target path, e.g. for the examination of small parts of the object, which may in particular be small organs of the patient or other small-sized areas of interest. A large predefined first threshold value might be suitable for an overview ultrasonic scan, which shall give an impression of the general situation of the object.

The first warning signal may be generated as a visual signal, an acoustic signal, a haptic signal or a combination of those or any other signal, which may attract the attention of the operator. In particular, the first warning signal may also be displayed similar to a traffic light, for example displaying a green light, when the distance between the current position and the predefined target path is low compared to the first threshold value. The traffic light may change to an orange light as soon as the distance is close to the first threshold value, for example at 90% of the first threshold value. As soon as the distance exceeds the first threshold, the traffic light may change to a red light.

The first warning signal may be generated by the display device, by the ultrasonic probe or by any other part of the ultrasonic instrument or by a different device, which might be connected via a communication interface to the ultrasonic instrument, such as a smartphone or any other communication device.

In some embodiments, the first warning signal, in particular the content of the first warning signal, may depend on the distance of the current position to the target path and may include an indication to guide the operator back to the predefined target path. This may also include an instruction to repeat parts of the path actually travelled or of parts of the predefined target path or other necessary parts of the object.

An advantage of such embodiments is that the attention of the operator may be attracted towards the predefined target path in the situation that the path has been left unintentionally. A risk of an undetected incorrect operation may be mitigated and the hit rate of a successful examination may be raised. In addition, novice operators might be able to perform less known examinations with better results.

According to several implementations, a second warning signal for the operator is generated, if the imaging data contains an artifact, a signal-to-noise ratio, which is less than a predefined minimum signal-to-noise ratio, and/or another image error.

The second warning signal may for example be generated if the imaging data contains an artifact, like a black bar, horizontal or vertical stripes or other artifacts. The second warning signal may also be generated, if the imaging data contains a signal-to-noise ratio, which is less than a predefined minimum signal-to-noise ratio. Another image error might for example be the missing black frame around the area of interest in a geometric shape of a cone or a frustum or another deviation from an expected regular imaging result. If the cone or the frustum is not fully developed, it might be an indication for an incorrect handling e.g. of the ultrasonic probe or an incorrect setting of the ultrasonic instrument.

The second warning signal may for example include information on how to avoid the incorrect handling or information on how to adjust the settings. For example, it is possible, that a currently applied pressure of the ultrasonic probe on the object is not a preferable range. If the currently applied pressure is too high or too low, artifacts or other image errors may occur.

The currently applied pressure of the ultrasonic probe might have an influence on the quality level of the imaging data. The currently applied pressure may change a potential penetration depth but may also change a current position of a part of the object, in particular in case of a patient, may change the current position e.g. of organs, air accumulations or other parts of the object. The currently applied pressure may be measured by at least one sensor, e.g. at least one tactility sensor or any other sensor for determining the currently applied pressure. The second warning signal may include information on how to avoid the artifact by changing the currently applied pressure in a better range.

During the ultrasonic scan, a coupling medium may be used between the ultrasonic transducer and the object, in particular, if the object is a patient, a skin, to displace air from the transducer-skin interface. This coupling medium may be referred to as ultrasonic transmission gel. In addition, the ultrasonic transmission gel may act as a lubricant, which enables smooth scanning performance.

It is possible, that an image error occurs due to fact that the currently applied amount of ultrasonic transmission gel is not sufficient. A propagation behavior of an ultrasonic beam may be significantly higher in a medium, which consists mainly of a liquid, such as water. An attenuation of the ultrasonic beam in a gaseous medium is very high. Thus, any part of the object or the transition of the ultrasonic transducer and the object, which includes gas or air might significantly reduce the quality level of the imaging data. Areas with a reduced amount of currently applied ultrasonic transmission gel might be visualized as black spots, distortions or other unintended image interferences. The imaging data may be evaluated according to such interferences, especially when evaluating a sequence of the ultrasonic images, the effect of missing ultrasonic transmission gel might be obviously visible. The evaluation may trigger the second warning signal accordingly. The second warning signal might include instructions for the operator to change the amount of ultrasonic transmission gel, in particular to increase the amount e.g. on specific parts of the ultrasonic transducer.

The second warning signal may be generated as a visual signal, an acoustic signal, a haptic signal or a combination of those or any other signal, which may attract the attention of the operator. In particular, the second warning signal may also be displayed similar to a traffic light, for example displaying a green light, when the imaging data does not include artifacts, low signal-to-noise ration and/or another image error. The traffic light may change to an orange light as soon as for example the signal-to-noise ratio approaches the predefined minimum signal-to-noise ratio, for example at 110% of the minimum signal-to-noise ratio. As soon as the signal-to-noise ratio falls below the predefined minimum signal-to-noise ratio, the traffic light may change to a red light. Same applies to a number of artifacts or the number of another image errors.

The second warning signal may be generated by the display device, by the ultrasonic probe or by any other part of the ultrasonic instrument or by a different device, which might be connected via a communication interface to the ultrasonic instrument, such as a smartphone or any other communication device.

An advantage of such embodiments is that the attention of the operator may be attracted to an incorrect operation regarding the imaging data. The novice operator might not be able to judge, if the current ultrasonic examination is leading to evaluable results. The second warning signal may give valuable guidance in a real-time scenario. This may mitigate the risk of having to repeat the ultrasonic scan at a later stage, if the fault is detected at a later stage e.g. by the data assessor.

According to several implementations, a third warning signal for the operator is generated, if a deviation between an examination parameter of the ultrasonic scan and a predefined reference value is larger than a predefined second threshold value, wherein the examination parameter is monitored during the ultrasonic scan.

In other words, the third warning signal may indicate a difference between the predefined reference values for the ultrasonic scan and the examination parameters. In particular, this might be an indication for an incorrect handling or an incorrect setting of the ultrasonic instrument. The examination parameters might be monitored by the ultrasonic instrument and might be compared to the individual predefined reference values in real-time.

The second threshold value may depend on the experience level of the operator or the examination scenario or other factors.

The third warning signal may be generated as a visual signal, an acoustic signal, a haptic signal or a combination of those or any other signal, which may attract the attention of the operator. In particular, the third warning signal may also be displayed similar to a traffic light, for example displaying a green light, when the deviation of the examination parameter and predefined reference value is low. The traffic light may change to an orange light as soon as for example the examination parameter approaches the predefined reference value, for example at 90% of the predefined reference value. As soon the examination parameter exceeds the predefined reference value, the traffic light may change to a red light.

The third warning signal may be generated by the display device, by the ultrasonic probe or by any other part of the ultrasonic instrument or by a different device, which might be connected via a communication interface to the ultrasonic instrument, such as a smartphone or any other communication device.

An advantage of such embodiment is that the attention of the operator may be attracted to an incorrect operation regarding the examination parameter. The third warning signal increases the assistance of the operator and may reduce a necessary time for performing the ultrasonic examination.

According to several implementations, the examination parameter is given by a current elevation angle of the ultrasonic probe or a current azimuth angle of the ultrasonic probe or a current output power of the ultrasonic probe or a current probe frequency of the ultrasonic probe or a current focus value of the ultrasonic probe or a current gain value of the ultrasonic probe.

The examination parameter may also be given by a combination of said quantities or may be derived from one or more of said quantities.

In other words, the examination parameter of the ultrasonic scan may for example be given by an orientation of the ultrasonic probe relative to the object in a Cartesian coordinate system, which may include the current elevation angle and the current azimuth angle. In addition, the examination parameter may be given by current settings of the ultrasonic probe, which may include the current output power, the current probe frequency, the current focus value or the current gain value.

As the ultrasonic probe may generate imaging data in a line-of-sight direction of the ultrasonic transducer, the current elevation angle of the ultrasonic probe and the current azimuth angle of the ultrasonic probe may be important degrees of freedom for a successful examination e.g. meaning to receive the imaging data that are expected. Small changes of the current elevation angle or of current the azimuth angle may have significant consequences on the imaging data in the ultrasonic examination. Both the current elevation angle and the current azimuth angle may be measured by at least one sensor e.g. at least one accelerometer, at least one gyroscope or any other sensor for determining changes of position.

The ultrasonic instrument might offer several adjustments for setting up measurement parameters. These measurement parameters may include the current output power, the current probe frequency, the current focus value, the current gain value and other relevant parameters. As the ultrasonic examination is based on a reflection or a transmission behavior of the object, a transmitted ultrasonic signal might change with a different output power or different frequency.

The current output power may influence the quality level of the imaging data. Different media may have different attenuation behavior and might require different ranges of the current output power. The third warning signal may instruct the operator to a most suitable value for the current output power for the area of interest.

The current frequency may influence the penetration depth and high frequencies may for example be superior if the area of interest is close to the surface of the object, low frequencies may for example be superior if the area of interest is located further away from the surface. In the case that the object is a patient, higher frequencies may for example be used for visualization of body parts close to a skin, while lower frequencies may for example be used for visualization of organs or other body parts, which are located further in the center of the body. A typical current frequency of the ultrasonic examination may be between for example 2 MHz and 20 MHz. The third warning signal may instruct the operator to the most suitable frequency for the area of interest.

The current focus value may change the width of an ultrasonic beam for example in a selected depth of the object. Adjusting the focus value may improve a spatial resolution in the area of interest as the width of the ultrasonic beam may be converged. A reduction of the width of the ultrasonic beam at the selected depth may be achieved at an expense of a deterioration in the width at other depths, resulting in poorer imaging data below a focus zone. The third warning signal may instruct the operator to change the current focus value to improve the imaging data at the selected depth of the object.

The current gain value may change an intensity of the reflected ultrasonic beam at a receiver for example for a further selected depth of the object. The current gain value may be a ratio of the current output power to a current input power. The current gain value may control a brightness of the imaging data in the further selected depth. In some embodiments, the method of adjusting the current gain value might be referred to as autogain. The third warning signal may instruct the operator to change the current gain value to improve the imaging data at the further selected depth of the object.

An advantage of such embodiments is a specific guidance of the operator to a relevant adjustment during the ultrasonic examination. The novice operator may be instructed towards an improved positioning of the ultrasonic probe and setting of the ultrasonic instrument and might be able to perform the ultrasonic examination faster and with improved results.

According to several implementations, sensor data determined by at least one accelerometer of the ultrasonic probe is received, for example from the ultrasonic probe, and the current position is determined based on the sensor data.

In other words, the at least one accelerometer may provide sensor data, which lead to a determination of the current position of the ultrasonic probe. The at least one accelerometer might be an integrated part of the ultrasonic probe or might be included elsewhere in the ultrasonic instrument, for example fixed to the hand of the operator holding the ultrasonic probe. Alternatively, the sensor data might be determined by at least one gyroscope or any other sensor for determining a change of position and/or orientation.

An advantage of such embodiments is the direct feedback of the current position of the ultrasonic probe to the operator by a particularly precise measurement.

According to several implementations, the current elevation angle of the ultrasonic probe and/or to the current azimuth angle of the ultrasonic probe is determined based on the sensor data. Alternatively, further sensor data determined by the at least one accelerometer is received, in particular from the ultrasonic probe, and the current elevation angle of the ultrasonic probe and/or to the current azimuth angle of the ultrasonic probe is determined based on the further sensor data.

In other words, the at least one accelerometer may provide further sensor data, which lead to a determination of the current elevation angle and/or of the current azimuth angle of the ultrasonic probe. Alternatively, the further sensor data might be determined by at least one gyroscope or any other sensor for determining a change of position and/or orientation.

An advantage of such embodiments is the direct feedback of at least one value mentioned above to the operator by a precise measurement. An improvement of a positioning of the ultrasonic probe may lead to an increased quality level of the imaging data.

According to several implementations, the at least one accelerometer comprises three accelerometers corresponding to three different spatial directions

Sensor data and/or further sensor data may be received by three accelerometers, each of them corresponding to one of the three different spatial directions. The three accelerometers might for example be positioned in different positions or in different parts of the ultrasonic probe or the ultrasonic instrument. Such implementations may lead to a higher precision of the sensor data and/or the further sensor data.

An advantage of such embodiments is the increased precision of the determination of the sensor data and/or of the further sensor data. As an information may be redundant, this may lead to a higher reliability and/or improve the lifetime of the ultrasonic instrument.

According to a further aspect of the invention, a data processing system is presented, which is configured to carry out a computer-implemented method for assisting an ultrasonic examination of an object according to the invention.

In the present disclosure, the expressions "data processing system" and "at least one data processing device" may be used interchangeably. A data processing device may in particular be understood as a data processing device, which comprises processing circuitry. The data processing device may therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, an ultrasonic instrument for an assisted ultrasonic examination of an object is presented, the ultrasonic instrument comprising a data processing system according to the invention, the ultrasonic probe and the display device.

The ultrasonic probe is configured to generate imaging data during an ultrasonic scan comprising a sequence of ultrasonic images generated by the ultrasonic probe and depicting the object. The data processing system is configured to monitor a current position of the ultrasonic probe during the ultrasonic scan. The display device is configured to display the current position of the ultrasonic probe and a predefined target path for the ultrasonic probe during the ultrasonic scan to guide an operator of the ultrasonic probe. In particular, the data processing system is configured to control the display device to display the current position and the and a predefined target path. The data processing system is configured to classify the imaging data by applying a trained machine learning model, MLM, to input data depending on the imaging data.

According to several implementations, the display device is configured to display an overlaid view comprising of the current position of the ultrasonic probe, the predefined target path and a completed path.

According to several implementations, the ultrasonic probe comprises a wireless ultrasonic probe.

In other words, it is possible that the ultrasonic probe is not attached to other parts of the ultrasonic instrument or any other part with a wired connection, in particular during the ultrasonic scan. The wireless ultrasonic probe may include a battery. The wireless ultrasonic probe may be connected e.g. to a power adapter for charging the battery after completion of the ultrasonic scan. The battery might be a rechargeable battery or a non-rechargeable battery.

An advantage of such embodiments is the flexibility of use for an operator during the ultrasonic examination. The operator may be occupied with different equipment during the ultrasonic scan and may not have additional support by other personnel during the ultrasonic examination. The assisted examination with the wireless ultrasonic probe might lead to a faster result and might reach a part of the object, which would e.g. not be reachable with a wired ultrasonic probe.

According to several implementations, the ultrasonic probe comprises by at least one accelerometer, which is configured to generate sensor data during the ultrasonic scan. The ultrasonic probe Is configured to transmit the sensor data to the data processing system. The data processing system is configured to determine the current position based on the sensor data.

Further implementations of the ultrasonic instrument according to the invention follow directly from the various embodiments of the computer-implemented methods according to the invention and vice versa. In particular, individual features and corresponding explanations as well as advantages relating to the various implementations of the computer-implemented methods according to the invention can be transferred analogously to corresponding implementations of the ultrasonic instrument according to the invention. In particular, the ultrasonic instrument according to the invention is designed or programmed to carry out a computer-implemented method according to the invention. In particular, the ultrasonic instrument according to the invention carries out a computer-implemented method according to the invention.

According to a further aspect of the invention, a computer program product is presented comprising instructions, which, when executed by an ultrasonic instrument cause a data processing system to carry out a computer-implemented method according to the invention.

The computer program, the further computer program and the computer-readable storage medium are respective computer program products comprising the instructions and/or the further instructions.

According to a further aspect of the invention, a method for ultrasonic-based medical diagnosis is provided. Therein, a computer-implemented method for assisting an ultrasonic examination of an object according to the invention is carried out, wherein the object is a patient. A medical diagnosis is made based on a result of the classification of the imaging data, for example automatically or in part automatically.

Further features and feature combinations of the invention are obtained from the figures and their description as well as the claims. In particular, further implementations of the invention may not necessarily contain all features of one of the claims. Further embodiments of the inventions may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,

FIG 1     shows a schematic flow diagram of an exemplary implementation of a computer-implemented method for an assisted ultrasonic examination according to the invention; and

FIG 2     shows a schematic flow diagram of a further exemplary implementation of a computer-implemented method for an assisted ultrasonic examination according to the invention; and

FIG 3     shows schematically an aspect of a further exemplary implementation of a computer-implemented method for an assisted ultrasonic examination; and

FIG 4     shows schematically an exemplary implementation of an ultrasonic instrument according to the invention;

FIG 5     shows schematically an artificial neural network; and

FIG 6     shows schematically a convolutional neural network.

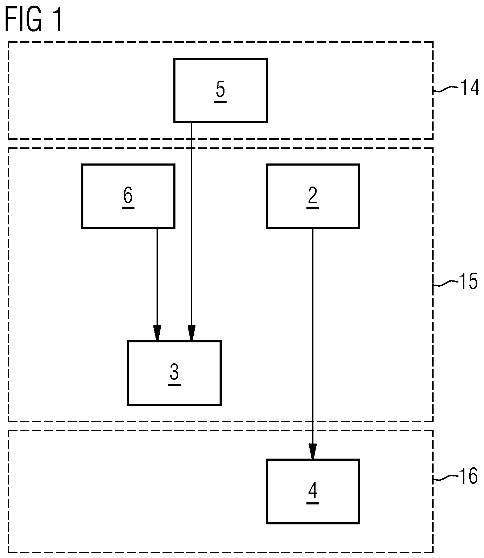

FIG 1 shows a schematic flow diagram of an exemplary implementation of the computer-implemented method for an assisted ultrasonic examination of an object 8 according to the invention. During an ultrasonic scan 15 with an ultrasonic probe 19, imaging data 2 comprising a sequence of ultrasonic images generated by the ultrasonic probe 19 and depicting the object 8 is received. A current position 6 of the ultrasonic probe 19 is monitored during the ultrasonic scan 15. The current position 6 of the ultrasonic probe 19 and a predefined target path 5 for the ultrasonic probe 19 are displayed during the ultrasonic scan 15 on a display device 3 to guide an operator of the ultrasonic probe 19. The imaging data 2 is classified by applying a trained machine learning model, MLM, to input data depending on the imaging data 2.

For example, an examination scenario may be selected in a predefinition phase 14. In the predefinition phase 14, the operator may prepare an ultrasonic instrument 23 for example by taking a picture of the object 8, in particular a picture of a patient, e.g. of the relevant body part of the patient. Additionally, the target path 5 may be defined in the predefinition phase 14, for example as a part of the examination scenario. The target path 5 may then be displayed on the display device 3. The picture of the object 8 may be displayed on the display device 3, for example as a background outline in particular in an overlaid view, so that the operator may see the target path 5 true to scale in combination with the object 8.

During the ultrasonic scan 15 the current position 6 of the ultrasonic probe 19 may be monitored and displayed on the display device 3. The current position 6 may be displayed on the display device 3 for example in the overlaid view as a position marker on the background outline of the object 8. The position marker of the current position 6 may move in real-time with the movement of the ultrasonic probe 19.

The imaging data 2 may be monitored and recorded during the ultrasonic scan 15. The imaging data 2 may serve as a feedback for the operator and may need to be available during the ultrasonic scan 15. The imaging data 2 may be displayed on the display device 3 in a different section of the display device 3, or maybe displayed on a different unit.

After completion of the ultrasonic scan 15, the imaging data 2 may be stored and ready to be processed by a data processing system 25 for classification 4 during a post-processing phase 16. The result of the classification 4 in combination with imaging data 2 might be a goal of the ultrasonic examination and may be made available to the operator or an assessor.

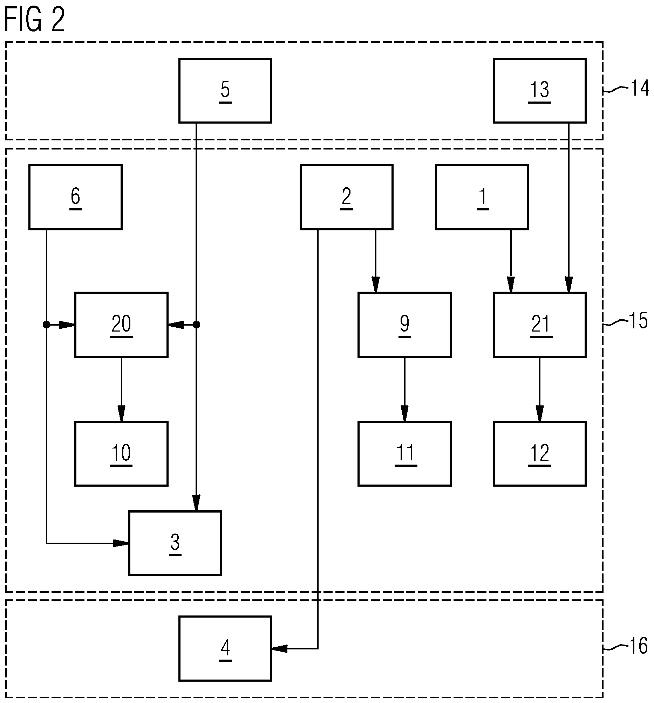

FIG 2 shows a schematic flow diagram of a further exemplary implementation of the computer-implemented method for an assisted ultrasonic examination of an object 8 according to the invention. The embodiment shown in FIG 2 may - unless otherwise stated - have all the features of FIG 1 by analogy.

The display device 3 may additionally display a completed path 7. The completed path 7 may for example be generated by connecting the current position 6 of the ultrasonic probe 19 with the previous positions of the ultrasonic probe 19, which have been monitored during the ultrasonic scan 15. The completed path 7 may be displayed next to the target path 5, in particular in the overlaid view, for example in a different color, different line shape or other distinguishing property. It is also possible that a portion of the target path 5 corresponding to the completed path 7 is displayed in a different color and/or format as the remainder of the target path 5 to indicate the completed path 7.

The current position 6 may be compared with the target path 5 during the ultrasonic scan 15 and a difference may be calculated. If the difference exceeds a predefined first threshold value 20, a first warning signal 10 may be generated. This first warning signal 10 may be displayed on the display device 3 and/or on a different device available to the operator. The first warning signal 10 may also be or include a different type of output, for example an acoustical output.

An evaluation 9 of the imaging data 2 may be performed during the ultrasonic scan 15. The evaluation 9 may include a check of the content of the imaging data 2 for artifacts, a level of a signal-to-noise ratio and/or another image error. The evaluation 9 may also include further checks, for example for the presence of an expected part of the object, in particular the part that should be seen for the selected examination scenario at the current position 6 of the ultrasonic probe 19. If the evaluation 9 reveals an abnormality, a second warning signal 11 may be generated. This second warning signal 11 may be displayed on the display device 3 and/or on a different device available to the operator. The second warning signal 11 may also be or include a different type of output, for example an acoustical output.

A reference value 13 may be defined in the predefinition phase 14, The reference value might include several numbers, which are relevant in the predefined examination scenario, e.g. reference value 13 of a setting of the ultrasonic instrument or reference value 13 for an orientation of the ultrasonic probe 19.

During the ultrasonic scan 15, an examination parameter 1 may be monitored. The examination parameter 1 may include a current elevation angle of the ultrasonic probe, a current azimuth angle of the ultrasonic probe, a current output power of the ultrasonic probe, a current probe frequency of the ultrasonic probe, a current focus value of the ultrasonic probe and a current gain value of the ultrasonic probe. During the ultrasonic scan 15, the examination parameter 1 may be monitored and compared with the reference value 13. In case the examination parameter 1 shows a deviation from the reference value 13, which is greater than a second threshold value 21, a third warning signal 12 may be generated. This third warning signal 12 may be displayed on the display device 3 and/or on a different device available to the operator. The third warning signal 10 may also be or include a different type of output, for example an acoustical output.

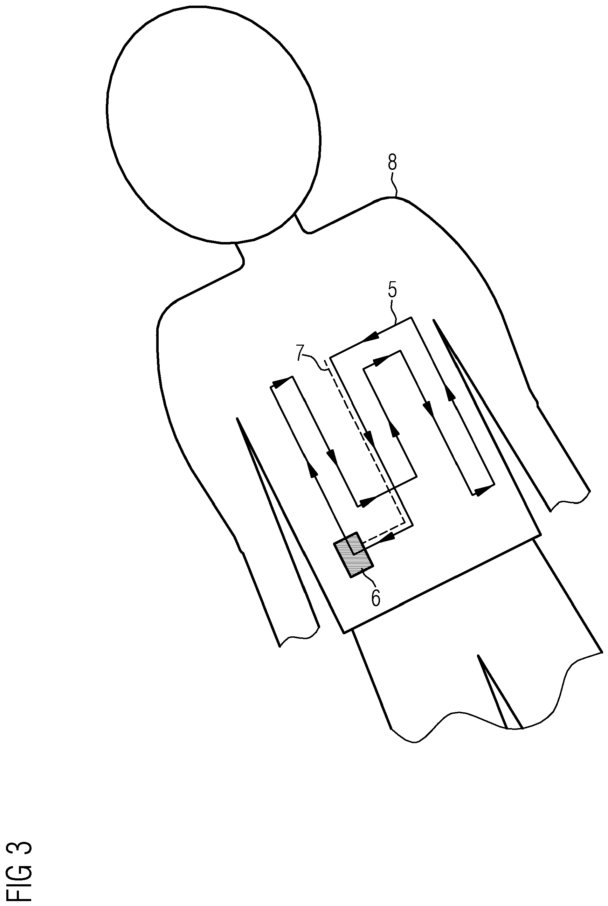

FIG 3 shows schematically an aspect of a further exemplary implementation of a computer-implemented method for an assisted ultrasonic examination. FIG 3 in particular shows the exemplary implementation with an overlaid view of the object 8, in particular of a patient. The patient may be displayed as a background outline of the picture of the specific person, or of a part of the specific person. Alternatively, a standard background outline may be displayed.

The preselected target path 5 may be displayed in a character layer further ahead of the picture of the object 8. The predefined target path 5 may feature arrows in order to highlight a direction of movement for the ultrasonic probe 19. The current position 6 of the ultrasonic probe 19 may be shown as symbol of the ultrasonic probe 19, in particular in real-time. The operator may receive direct feedback after moving the ultrasonic probe 19.

The completed path 7 may be displayed as an overlaid view in a further character layer further ahead of the target path 5 for example as a dashed or dotted line or a line in a different color or a line with any other distinguishing feature. Alternatively a part of the target path 5 may appear in a different color once the completed path 7 is congruent with it. This may leave a part of the target path 5, which has not yet been scanned, in an original color. Thus, the operator may get an indication of the progress of the ultrasonic examination.

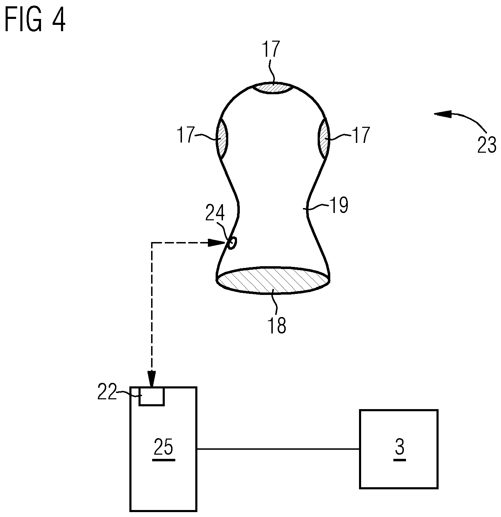

FIG 4 shows schematically an exemplary implementation of an ultrasonic instrument 23 according to the invention. The ultrasonic instrument 23 includes an ultrasonic probe 19, which may include an ultrasonic transducer 18 and at least one accelerometer 17. The imaging data 2 may be generated in the perspective of the ultrasonic transducer 18. FIG 4 shows an example of an implementation with three accelerometers 17 in three different positions corresponding to the three spatial directions. The accelerometers 17 may also be positioned closer together, for example next to each other. It is noted that, in alternative implementations, the ultrasonic probe 19 does not necessarily comprise the at least one accelerometer 17 for monitoring the current position 6. Rather, different types of monitoring systems may be provided including, for example, a camera provided separately to the ultrasonic probe 19.

The ultrasonic instrument 23 includes a data processing system 25 comprising a communication unit 22. The communication unit 22 may be configured for bidirectional data exchange with a communication adapter 24, which may be part of the ultrasonic probe 19. The type of communication between the communication unit 22 and the communication adapter 24 may be wired communication or wireless communication.

The ultrasonic instrument 23 may also include a display device 3, which is connected to the data processing system 25. The type of communication between the data processing system 25 and the display device may be wired communication or wireless communication. The data processing system 25 may be configured to control the display device 3 and to compute the relevant information and send to the display device 3, including for example the target path 5, the completed path 7, the first warning signal 10, the second warning signal 11 and the third warning signal 12.

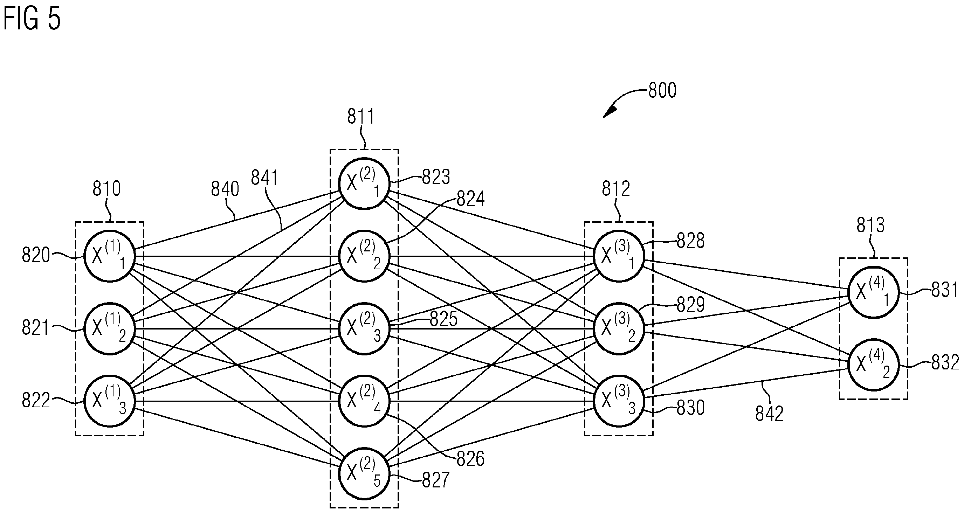

The MLM may for example be an artificial neural network, ANN. FIG 5 displays an embodiment of an ANN 800, which is for example designed as an MLP. The ANN 800 comprises nodes 820, ..., 832 and edges 840, ..., 842, wherein each edge 840, ..., 842 is a directed connection from a first node 820, ..., 832 to a second node 820, ..., 832. In general, the first node 820, ..., 832 and the second node 820, ..., 832 are different nodes 820, ..., 832. It is, however, also possible that the first node 820, ..., 832 and the second node 820, ..., 832 are identical. For example, in FIG 5, the edge 840 is a directed connection from the node 820 to the node 823, and the edge 842 is a directed connection from the node 830 to the node 832. An edge 840, ... , 842 from a first node 820, ..., 832 to a second node 820, ... , 832 is also denoted as ingoing edge for the second node 820, ..., 832 and as outgoing edge for the first node 820, ... , 832.

In this example, the nodes 820, ... , 832 of the artificial neural network 800 can be arranged in layers 810, ... , 813, wherein the layers can comprise an intrinsic order introduced by the edges 840, ... , 842 between the nodes 820, ... , 832. In particular, edges 840, ... , 842 can exist only between neighboring layers of nodes. In the displayed example, there is an input layer 810 comprising only nodes 820, ... , 822 without an incoming edge, an output layer 813 comprising only nodes 831, 832 without outgoing edges, and hidden layers 811, 812 in-between the input layer 810 and the output layer 813. In general, the number of hidden layers 811, 812 can be chosen arbitrarily. In an MLP, this number is at least one. The number of nodes 820, ..., 822 within the input layer 810 usually relates to the number of input values of the artificial neural network 800, and the number of nodes 831, 832 within the output layer 813 usually relates to the number of output values of the artificial neural network 800.

In particular, a real number can be assigned as a value to every node 820, ... , 832 of the artificial neural network 800. Here, $x^{(n)}_i$ denotes the value of the i-th node 820, ... , 832 of the n-th layer 810, ... , 813. The values of the nodes 820, ... , 822 of the input layer 810 are equivalent to the input values of the artificial neural network 800. The values of the nodes 831, 832 of the output layer 813 are equivalent to the output value of the artificial neural network 800. Furthermore, each edge 840, ... , 842 can comprise a weight being a real number. In particular, the weight is a real number within the interval [-1, 1] or within the interval [0, 1]. Here, $w^{(m,n)}_{i,j}$ denotes the weight of the edge between the i-th node 820, ... , 832 of the m-th layer 810, ... , 813 and the j-th node 820, 832 of the n-th layer 810, ... , 813. Furthermore, the abbreviation $w^{(n)}_{i,j}$ is defined for the weight $w^{(n,n+1)}_{i,j}$. In particular, to calculate the output values of the neural network 800, the input values are propagated through the neural network 800. In particular, the values of the nodes 820, ... , 832 of the (n+1)-th layer 810, ... , 813 can be calculated based on the values of the nodes 820, ... , 832 of the n-th layer 810, ..., 813 by

$$x^{(n+1)}_j = f\left(\sum_i x^{(n)}_i \; w^{(n)}_{i,j}\right).$$

Herein, the function f is denoted as transfer function or activation function. Known transfer functions are step functions, the sigmoid functions, for example the logistic function, the generalized logistic function, the hyperbolic tangent, the arctangent function, the error function, the smoothstep function, or rectifier functions. The transfer function is for example used for normalization purposes. In particular, the values are propagated layer-wise through the neural network 800, wherein values of the input layer 810 are given by the input of the neural network 800, wherein values of the first hidden layer 811 can be calculated based on the values of the input layer 810 of the neural network 800, wherein values of the second hidden layer 812 can be calculated based on the values of the first hidden layer 811, and so forth.

In order to set the values $w^{(m,n)}_{i,j}$ for the edges, the neural network 800 has to be trained using training data. In particular, training data comprises training input data and training output data (denoted as $t_i$). For a training step, the neural network 800 is applied to the training input data to generate calculated output data. In particular, the training data and the calculated output data comprise a number of values, said number being equal with the number of nodes of the output layer. In particular, a comparison between the calculated output data and the training data is used to recursively adapt the weights within the neural network 800 (backpropagation algorithm). In particular, the weights are changed according to

$$w'^{(n)}_{i,j} = w^{(n)}_{i,j} - \gamma \, \delta^{(n)}_j \, x^{(n)}_i,$$

wherein $\gamma$ is a predefined learning rate, and the numbers $\delta^{(n)}_j$ can be recursively calculated as

$$\delta^{(n)}_j = \left(\sum_k \delta^{(n+1)}_k \, w^{(n+1)}_{j,k}\right) f'\left(x^{(n)}_i w^{(n)}_{i,j}\right)$$

based on $\delta^{(n+1)}_j$, if the (n+1)-th layer is not the output layer 813, and

$$\delta^{(n)}_j = \left(x^{(n+1)}_j - t^{(n+1)}_j\right) \; f'\left(x^{(n)}_i w^{(n)}_{i,j}\right),$$

if the (n+1)-th layer is the output layer 813, wherein f' is the first derivative of the activation function, and $t^{(n+1)}_j$ is the comparison training value for the j-th node of the output layer 813.

The MLM may for example be a convolutional neural network, CNN. A convolutional neural network, CNN, is an ANN that uses a convolution operation instead of general matrix multiplication in at least one of its layers. These layers are denoted as convolutional layers. In particular, a convolutional layer performs a dot product of one or more convolution kernels with the convolutional layer's input data, wherein the entries of the one or more convolution kernel are parameters or weights that may be adapted by training. In particular, one can use the Frobenius inner product and the ReLU activation function. A convolutional neural network can comprise additional layers, for example pooling layers, fully connected layers, and/or normalization layers.

By using convolutional neural networks, the input can be processed in a very efficient way because a convolution operation based on different kernels can extract various image features so that by adapting the weights of the convolution kernel the relevant image features can be found during training. Furthermore, based on the weight-sharing in the convolutional kernels fewer parameters need to be trained, which prevents overfitting in the training phase and allows to have faster training or more layers in the network, improving the performance of the network.

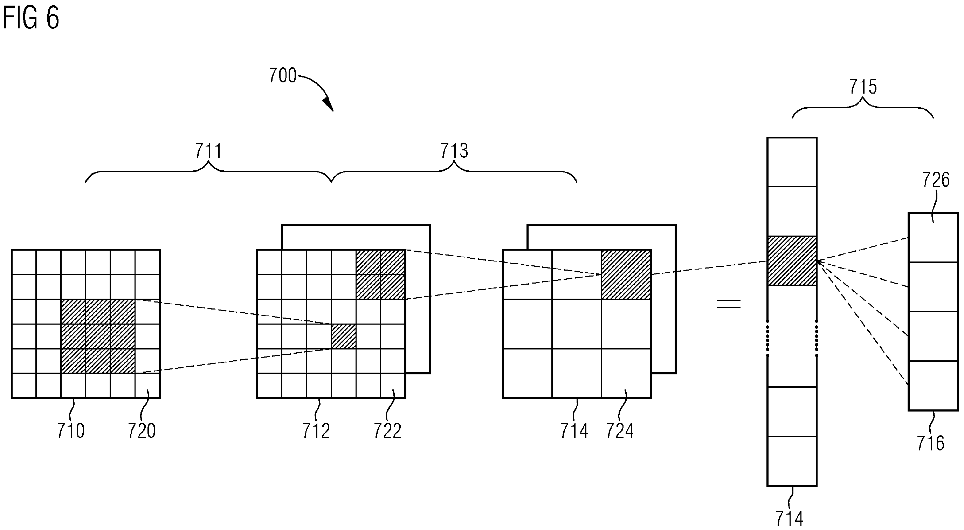

FIG 6 displays an exemplary embodiment of a convolutional neural network 700. In the displayed embodiment, the convolutional neural network 700 comprises an input node layer 710, a convolutional layer 711, a pooling layer 713, a fully connected layer 714 and an output node layer 716, as well as hidden node layers 712, 714. Alternatively, the convolutional neural network 200 can comprise several convolutional layers 711, several pooling layers 713 and/or several fully connected layers 715, as well as other types of layers. The order of the layers can be chosen arbitrarily, usually fully connected layers 715 are used as the last layers before the output layer 716.

In particular, within a convolutional neural network 700 nodes 720, 722, 724 of a node layer 710, 712, 714 can be considered to be arranged as a d-dimensional matrix or as a d-dimensional image. In particular, in the two-dimensional case the value of the node 720, 722, 724 indexed with i and j in the n-th node layer 710, 712, 714 can be denoted as x(n)[i, j]. However, the arrangement of the nodes 720, 722, 724 of one node layer 710, 712, 714 does not have an effect on the calculations executed within the convolutional neural network 700 as such, since these are given solely by the structure and the weights of the edges.

A convolutional layer 711 is a connection layer between an anterior node layer 710 with node values x(n-1) and a posterior node layer 712 with node values x(n). In particular, a convolutional layer 711 is characterized by the structure and the weights of the incoming edges forming a convolution operation based on a certain number of kernels. In particular, the structure and the weights of the edges of the convolutional layer 711 are chosen such that the values x(n) of the nodes 722 of the posterior node layer 712 are calculated as a convolution x(n) = K * x(n-1) based on the values x(n-1) of the nodes 720 anterior node layer 710, where the convolution * is defined in the two-dimensional case as

$$x^{(n)}[i,j] = \left(K * x^{(n-1)}\right)[i,j] = \sum_{i'}\sum_{j'} K[i',j'] \cdot x^{(n-1)}[i-i', j-j'].$$

Herein, the kernel K is a d-dimensional matrix, in the present example a two-dimensional matrix, which is usually small compared to the number of nodes 720, 722, for example a 3x3 matrix, or a 5x5 matrix. In particular, this implies that the weights of the edges in the convolution layer 711 are not independent, but chosen such that they produce said convolution equation. In particular, for a kernel being a 3x3 matrix, there are only 9 independent weights, each entry of the kernel matrix corresponding to one independent weight, irrespectively of the number of nodes 720, 722 in the anterior node layer 710 and the posterior node layer 712.

In general, convolutional neural networks 700 use node layers 710, 712, 714 with a plurality of channels, in particular, due to the use of a plurality of kernels in convolutional layers 711. In those cases, the node layers can be considered as (d+1)-dimensional matrices, the first dimension indexing the channels. The action of a convolutional layer 711 is then in a two-dimensional example defined as

$$x_b^{(n)}[i,j] = \sum_a (K_{a,b} * x_a^{(n-1)}[i,j] = \sum_a\sum_{i'}\sum_{j'} K_{a,b}[i',j'] \cdot x_a^{(n-1)}[i-i', j-j'],$$

wherein $x_a^{(n)}$ corresponds to the a-th channel of the anterior node layer 710, $x_b^{(n)}$ corresponds to the b-th channel of the posterior node layer 712 and $K_{a,b}$ corresponds to one of the kernels. If a convolutional layer 711 acts on an anterior node layer 710 with A channels and outputs a posterior node layer 712 with B channels, there are A·B independent d-dimensional kernels $K_{a,b}$.

In general, in convolutional neural networks 700 activation functions may be used. In this embodiment, ReLU (rectified linear unit) is used, with R(z) = max(0, z), so that the action of the convolutional layer 711 in the two-dimensional example is

$$x_b^{(n)}[i,j] = R\left(\sum_a \left(K_{a,b} * x_a^{(n-1)}[i,j]\right)\right) = R\left(\sum_a \sum_{i'} \sum_{j'} K_{a,b}[i',j'] \cdot x_a^{(n-1)}[i-i',j-j']\right).$$

It is also possible to use other activation functions, for example ELU (exponential linear unit), LeakyReLU, Sigmoid, Tanh or Softmax.

In the displayed embodiment, the input layer 710 comprises 36 nodes 720, arranged as a two-dimensional 6x6 matrix. The first hidden node layer 712 comprises 72 nodes 722, arranged as two two-dimensional 6x6 matrices, each of the two matrices being the result of a convolution of the values of the input layer with a 3x3 kernel within the convolutional layer 711. Equivalently, the nodes 722 of the first hidden node layer 712 can be interpreted as arranged as a three-dimensional 2x6x6 matrix, wherein the first dimension correspond to the channel dimension.

An advantage of using convolutional layers 711 is that spatially local correlation of the input data can exploited by enforcing a local connectivity pattern between nodes of adjacent layers, in particular by each node being connected to only a small region of the nodes of the preceding layer.

A pooling layer 713 is a connection layer between an anterior node layer 712 with node values x(n-1) and a posterior node layer 714 with node values x(n). In particular, a pooling layer 713 can be characterized by the structure and the weights of the edges and the activation function forming a pooling operation based on a non-linear pooling function f. For example, in the two-dimensional case the values x(n) of the nodes 724 of the posterior node layer 714 can be calculated based on the values x(n-1) of the nodes 722 of the anterior node layer 712 as

$$x_b^{(n)}[i,j] = f\left(x_b^{(n-1)}[id_1, jd_2], \dots, \quad x_b^{(n-1)}[(i+1)d_1 - 1, (j+1)d_2 - 1]\right).$$

In other words, by using a pooling layer 713, the number of nodes 722, 724 can be reduced by re-placing a number d1 d2 of neighboring nodes 722 in the anterior node layer 712 with a single node 722 in the posterior node layer 714 being calculated as a function of the values of said number of neighboring nodes. In particular, the pooling function f can be the max-function, the average or the L2-Norm. In particular, for a pooling layer 713 the weights of the incoming edges are fixed and are not modified by training.

The advantage of using a pooling layer 713 is that the number of nodes 722, 724 and the number of parameters is reduced. This leads to the amount of computation in the network being reduced and to a control of overfitting.

In the displayed embodiment, the pooling layer 713 is a max-pooling layer, replacing four neighboring nodes with only one node, the value being the maximum of the values of the four neighboring nodes. The max-pooling is applied to each d-dimensional matrix of the previous layer. In this embodiment, the max-pooling is applied to each of the two two-dimensional matrices, reducing the number of nodes from 72 to 18.

In general, the last layers of a convolutional neural network 700 may be fully connected layers 715. A fully connected layer 715 is a connection layer between an anterior node layer 714 and a posterior node layer 716. A fully connected layer 713 can be characterized by the fact that a majority, in particular, all edges between nodes 714 of the anterior node layer 714 and the nodes 716 of the posterior node layer are present, and wherein the weight of each of these edges can be adjusted individually.

In this embodiment, the nodes 724 of the anterior node layer 714 of the fully connected layer 715 are displayed both as two-dimensional matrices, and additionally as non-related nodes, indicated as a line of nodes, wherein the number of nodes was reduced for a better presentability. This operation is also denoted as flattening. In this embodiment, the number of nodes 726 in the posterior node layer 716 of the fully connected layer 715 smaller than the number of nodes 724 in the anterior node layer 714. Alternatively, the number of nodes 726 can be equal or larger.

Furthermore, in this embodiment the Softmax activation function is used within the fully connected layer 715. By applying the Softmax function, the sum the values of all nodes 726 of the output layer 716 is 1, and all values of all nodes 726 of the output layer 716 are real numbers between 0 and 1. In particular, if using the convolutional neural network 700 for categorizing input data, the values of the output layer 716 can be interpreted as the probability of the input data falling into one of the different categories.

In particular, convolutional neural networks 700 can be trained based on the backpropagation algorithm. For preventing overfitting, methods of regularization can be used, for example dropout of nodes 720, ... , 724, stochastic pooling, use of artificial data, weight decay based on the L1 or the L2 norm, or max norm constraints.

Independent of the grammatical term usage, individuals with male, female or other gender identities are included within the term.

**Claims**

1. Computer-implemented method for assisting an ultrasonic examination of an object (8), wherein

   - during an ultrasonic scan (15) with an ultrasonic probe (19), imaging data (2) comprising a sequence of ultrasonic images generated by the ultrasonic probe (19) and depicting the object (8) is received; and
   - a current position (6) of the ultrasonic probe (19) is monitored during the ultrasonic scan (15); and
   - the current position (6) of the ultrasonic probe (19) and a predefined target path (5) for the ultrasonic probe (19) are displayed during the ultrasonic scan (15) on a display device (3) to guide an operator of the ultrasonic probe (19); and
   - the imaging data (2) is classified by applying a trained machine learning model, MLM, to input data depending on the imaging data (2).

2. Computer-implemented method according to claim 1, wherein, depending on the current position (6), a completed path (7) for the ultrasonic probe (19) is displayed during the ultrasonic scan (15) on the display device (3).

3. Computer-implemented method according to one of the preceding claims, wherein a first warning signal (10) for the operator is generated, if a distance between the current position (6) and the predefined target path (5) exceeds a predefined first threshold value (20).

4. Computer-implemented method according to one of the preceding claims, wherein a second warning signal (11) for the operator is generated, if the imaging data (2) contains an artifact, a signal-to-noise ratio, which is less than a predefined minimum signal-to-noise ratio, and/or another image error.

5. Computer-implemented method according to one of the preceding claims, wherein a third warning signal (12) is generated, if a deviation between an examination parameter (1) of the ultrasonic scan (15) and a predefined reference value (13) is larger than a predefined second threshold value (21), wherein the examination parameter (1) is monitored during the ultrasonic scan (15).

6. Computer-implemented method according to claim 5, wherein the examination parameter (1) is given by

   - a current elevation angle of the ultrasonic probe (19) or
   - a current azimuth angle of the ultrasonic probe (19) or
   - a current output power of the ultrasonic probe (19) or
   - a current probe frequency of the ultrasonic probe (19) or
   - a current focus value of the ultrasonic probe (19) or
   - a current gain value of the ultrasonic probe (19).

7. Computer-implemented method according to one of the preceding claims, wherein sensor data determined by at least one accelerometer (17) of the ultrasonic probe (19) is received and the current position (6) is determined based on the sensor data.

8. Computer-implemented method according to claims 6 and 7, wherein

   - the current elevation angle of the ultrasonic probe (19) and/or to the current azimuth angle of the ultrasonic probe (19) is determined based on the sensor data or
   - further sensor data determined by the at least one accelerometer (17) is received and the current elevation angle of the ultrasonic probe (19) and/or to the current azimuth angle of the ultrasonic probe (19) is determined based on the further sensor data.

9. Computer-implemented method according to claims 7 or 8, wherein the at least one accelerometer (17) comprises three accelerometers (17) corresponding to three different spatial directions.

10. Data processing system (25), which is configured to carry out a computer-implemented method according to one of the preceding claims.

11. Ultrasonic instrument (23) for an assisted ultrasonic examination of an object (8) comprising a data processing system (25) according to claim 10, the ultrasonic probe (19) and the display device (3).

12. Ultrasonic instrument (23) according to claim 11, wherein the display device (3) is configured to display an overlaid view comprising of the current position (6) of the ultrasonic probe (19) and the target path (5).

13. Ultrasonic instrument (23) according to one of claims 11 or 12, wherein the ultrasonic probe (19) is a wireless ultrasonic probe.

14. Ultrasonic instrument (23) according to one of claims 11 to 13, wherein

   - the ultrasonic probe (19) comprises by at least one accelerometer (17), which is configured to generate sensor data during the ultrasonic scan (15);
   - the ultrasonic probe (19) is configured to transmit the sensor data to the data processing system (25); and
   - the data processing system (25) is configured to determine the current position (6) based on the sensor data.

15. Computer program product comprising instructions, which, when executed by a data processing system (25), cause the data processing system (25) to carry out a computer-implemented method according to one of claims 1 to 9.

FIG 1

FIG 2

FIG 3

FIG 4

FIG 5

EP 4 666 955 A1

FIG 6

Europäisches
Patentamt
European
Patent Office
Office européen
des brevets

# EUROPEAN SEARCH REPORT

**Application Number**

EP 24 18 3366

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/289094 A1 (DE JONGE MATTHEW [US] ET AL) 17 September 2020 (2020-09-17) | 1,2,4-15 | INV. A61B8/00 |
| Y | * figures 1,2,3B * | 3 | A61B8/08 |
| | * paragraph [0162] * | | |
| | * paragraph [0184] * | | |
| | * paragraph [0186] - paragraph [0187] * | | |
| | * paragraph [0190] * | | |
| | * paragraph [0193] * | | |
| | * paragraph [0196] * | | |
| | * paragraph [0237] * | | |
| | * paragraph [0260] * | | |
| | * paragraph [0314] * | | |
| | ----- | | |
| Y | CN 109 646 047 A (SHANGHAI QIANCONG NETWORK TECH CO LTD) 19 April 2019 (2019-04-19) * claims 1,8 * | 3 | |
| | ----- | | |
| Y | US 2017/215842 A1 (RYU JAE-YOUNG [KR] ET AL) 3 August 2017 (2017-08-03) * figure 8B * * paragraph [0130] - paragraph [0134] * | 1,10,15 | **TECHNICAL FIELDS SEARCHED (IPC)** |
| | ----- | | A61B |
| Y | US 2022/386990 A1 (ROTHBERG ALEX [US]) 8 December 2022 (2022-12-08) * figures 1,19 * * paragraph [0060] * * paragraph [0116] * | 1,10,15 | |
| | ----- | | |

The present search report has been drawn up for all claims

| Place of search | Date of completion of the search | Examiner |
|---|---|---|
| Munich | 29 October 2024 | Willig, Hendrik |

EPO FORM 1503 03.82 (P04C01)

## ANNEX TO THE EUROPEAN SEARCH REPORT
## ON EUROPEAN PATENT APPLICATION NO.

EP 24 18 3366

This annex lists the patent family members relating to the patent documents cited in the above-mentioned European search report.
The members are as contained in the European Patent Office EDP file on
The European Patent Office is in no way liable for these particulars which are merely given for the purpose of information.

29-10-2024

| Patent document cited in search report | | | Publication date | Patent family member(s) | | | Publication date |
|---|---|---|---|---|---|---|---|
| US 2020289094 | A1 | | 17-09-2020 | AU | 2017281281 | A1 | 06-12-2018 |
| | | | | AU | 2022203420 | A1 | 09-06-2022 |
| | | | | CA | 3026162 | A1 | 28-12-2017 |
| | | | | CN | 109310396 | A | 05-02-2019 |
| | | | | CN | 113876353 | A | 04-01-2022 |
| | | | | EP | 3471623 | A1 | 24-04-2019 |
| | | | | EP | 4201340 | A1 | 28-06-2023 |
| | | | | JP | 7361470 | B2 | 16-10-2023 |
| | | | | JP | 2019521745 | A | 08-08-2019 |
| | | | | JP | 2022123076 | A | 23-08-2022 |
| | | | | KR | 20190021344 | A | 05-03-2019 |
| | | | | TW | 201800057 | A | 01-01-2018 |
| | | | | US | 2017360401 | A1 | 21-12-2017 |
| | | | | US | 2017360402 | A1 | 21-12-2017 |
| | | | | US | 2017360403 | A1 | 21-12-2017 |
| | | | | US | 2017360404 | A1 | 21-12-2017 |
| | | | | US | 2017360411 | A1 | 21-12-2017 |
| | | | | US | 2017360412 | A1 | 21-12-2017 |
| | | | | US | 2020289094 | A1 | 17-09-2020 |
| | | | | US | 2021052249 | A1 | 25-02-2021 |
| | | | | US | 2021275145 | A1 | 09-09-2021 |
| | | | | US | 2022167945 | A1 | 02-06-2022 |
| | | | | US | 2023117915 | A1 | 20-04-2023 |
| | | | | US | 2024161491 | A1 | 16-05-2024 |
| | | | | WO | 2017222970 | A1 | 28-12-2017 |
| CN 109646047 | A | | 19-04-2019 | NONE | | | |
| US 2017215842 | A1 | | 03-08-2017 | CA | 2958281 | A1 | 03-03-2016 |
| | | | | CN | 106659474 | A | 10-05-2017 |
| | | | | EP | 3185778 | A1 | 05-07-2017 |
| | | | | JP | 6683677 | B2 | 22-04-2020 |
| | | | | JP | 2017531455 | A | 26-10-2017 |
| | | | | KR | 20160025891 | A | 09-03-2016 |
| | | | | US | 2017215842 | A1 | 03-08-2017 |
| | | | | WO | 2016032298 | A1 | 03-03-2016 |
| US 2022386990 | A1 | | 08-12-2022 | AU | 2018323621 | A1 | 06-02-2020 |
| | | | | CA | 3070582 | A1 | 07-03-2019 |
| | | | | EP | 3675727 | A1 | 08-07-2020 |
| | | | | TW | 201912114 | A | 01-04-2019 |
| | | | | US | 2019059851 | A1 | 28-02-2019 |
| | | | | US | 2022386990 | A1 | 08-12-2022 |
| | | | | WO | 2019046521 | A1 | 07-03-2019 |

EPO FORM P0459

For more details about this annex : see Official Journal of the European Patent Office, No. 12/82

**REFERENCES CITED IN THE DESCRIPTION**

**Patent documents cited in the description**

- KR 102097740 B1